# EUROPEAN PATENT SPECIFICATION

(11) **EP 2 915 475 B1**
(45) Date of publication and mention of the grant of the patent: **20.06.2018**
(21) Application number: 13850136.6
(22) Date of filing: 28.10.2013
(51) Int. Cl.: A61B 1/00, G02B 23/24

(54) **AUXILIARY INSERTION AND REMOVAL TOOL AND ENDOSCOPE HAVING THIS AUXILIARY INSERTION AND REMOVAL TOOL**
VORRICHTUNG ZUR UNTERSTÜTZUNG EINER EINSETZUNG UND ENTNAHME SOWIE ENDOSKOP MIT DIESER VORRICHTUNG ZUR UNTERSTÜTZUNG EINER EINSETZUNG UND ENTNAHME
DISPOSITIF D'AIDE À L'INSERTION ET À L'EXTRACTION ET ENDOSCOPE COMPRENANT CE DISPOSITIF D'AIDE À L'INSERTION ET À L'EXTRACTION

(30) Priority: 05.11.2012 JP 2012243462
(43) Date of publication of application: 09.09.2015
(73) Proprietor: Olympus Corporation, Tokyo 192-8507 (JP)
(72) Inventor: NISHIIE, Takehiro, Hachioji-shi, Tokyo 192-8507 (JP)
(74) Representative: Schicker, Silvia
(86) International application number: PCT/JP2013/079179
(87) International publication number: WO 2014/069424

(56) References cited:
- WO-A1-2006/109598
- WO-A1-2012/137361
- JP-A- 2005 329 000
- JP-A- 2006 218 235
- JP-A- 2008 119 218
- JP-A- 2008 272 302
- JP-A- 2011 520 563
- US-A- 4 630 649
- US-A1- 2009 023 994
- US-A1- 2011 319 713

## Description

### Technical Field

The present invention relates to an auxiliary insertion and removal device that aids insertion and removal of an inserting section into a lumen, and an endoscope having this auxiliary insertion and removal device.

### Background Art

For example, Patent Literature 1 discloses a rotary self-propelled endoscope that improves insertability into a body cavity and operability. The endoscope has an inserting section main body, a rotary cylindrical body rotatably arranged on an outer periphery of the inserting section main body, and a rotation driving means coupled with a proximal end portion of the rotary cylindrical body. A surface of the rotary cylindrical body has a spiral shape. The rotary driving means has a motor, a gear, and a rotary pipe. When the motor rotates, a rotary drive force is produced. This rotary drive force is transmitted to the entire rotary cylindrical body through the gear, the rotary pipe, and a proximal end portion of the rotary cylindrical body. As a result, the rotary cylindrical body rotates around an axis of the rotary cylindrical body.

For example, Patent Literature 2 discloses a rotary self-propelled endoscope that improves insertability of an inserting section. The endoscope has an inserting section and a motor. The inserting section has an inserting section main body and a rotary cylindrical body into which the inserting section main body is inserted. The rotary cylindrical body has a spiral-shaped portion formed on an outer peripheral surface of the rotary cylindrical body. When the motor imparts a rotary drive force to the rotary cylindrical body, the rotary cylindrical body can rotate around an axis of an inserting direction. Note that the motor imparts the rotary drive force to a proximal end portion side, an intermediate portion, or a distal end portion of the rotary cylindrical body.

Patent Literature 3 discloses a rotate-to-advance catheterization system including a threaded introducer catheter having features designed to maximize the tortional stiffness of its tube while minimizing bending stiffness of the same tube; for example, the tube may be formed with a composite construction comprising an inner convoluted or corrugated tube, with or without a braided fiber layer and with or without flexible outside layer, where the torsional and bending characteristics of the corrugated or convoluted tube may be optimized by varying the geometry and/or the material along the length of the device. One or more low friction bearings may be positioned within the catheter's interior lumen, so as to reduce surface contact with the endoscope, where the bearings include a protrusion which is adapted to ride in the helical through the convoluted or corrugated tube; alternatively, one or more low friction bearings may be provided, where the bearings include a recess for receiving the helical peak of the convoluted or corrugated tube. In another embodiment, a smooth liner, disposed within the internal diameter of the corrugated tube, is used so as to reduce friction when a visualization device or instrument is disposed within the tube; the liner may be composed of multiple layers (such as an elastic layer supporting a tow friction layer) to allow bending without kinking, and may employ a coating to reduce frictional drag or be composed of lubricant blended compound.

Patent Literature 4 discloses an endoscope including an attachment unit comprising a tube main body and a fin portion, where the tube main body includes a metal helical tube, a metal reticular tube that covers the outer peripheral direction side of the helical tube, and a resin envelope that covers the outer peripheral direction side of the reticular tube; the fin portion is made in resin and is integrally formed from the envelope of the tube main body.

### Citation List

### Patent Literature

Patent Literature 1: Jpn. Pat. Appln. KOKAI Publication No. 2008-119218
Patent Literature 2: Jpn. Pat. Appln. KOKAI Publication No. 2008-272302
Patent Literature 3: US Pat. Appln. Publication No. 2011/0319713 A1
Patent Literature 4: International Pat. Appln. Publication No. 2012/137361 A1

### Summary of Invention

### Technical Problem

In Patent Literature 1 and Patent Literature 2 described above, the rotary drive force is transmitted to only part of the rotary cylindrical body rather than the entire rotary cylindrical body, whereby the entire rotary cylindrical body rotates. Therefore, the inserting section
including the rotary cylindrical body moves forward and backward in a lumen. At this time, the rotary cylindrical body receives resistance from an inner wall of the lumen. In a state where the rotary drive force is transmitted to part of the rotary cylindrical body alone, when the rotary cylindrical body receives the resistance, there is fear that rotation of the rotary cylindrical body stops.

That is, in a state where the rotary drive force is transmitted to part of the rotary cylindrical body alone, the transmissibility of the rotary drive force to the rotary cylindrical body is lowered. Therefore, an improvement in transmissibility of the rotary drive force to the rotary cylindrical body, i.e., transmission of the rotary drive force to the entire rotary cylindrical body, is desired.

Therefore, in view of the above-described situation, it is an object of the present invention to provide an auxiliary insertion and removal device that can improve the transmissibility of the rotary drive force and an endoscope having this auxiliary insertion and removal device.

### Solution to Problem

The above object is achieved by the auxiliary insertion and removal device according to claim 1.

### Advantageous Effects of Invention

According to the present invention, it is possible to provide the auxiliary insertion and removal device that can improve the transmissibility of the rotary drive force and the endoscope having this auxiliary insertion and removal device.

### Brief Description of Drawings

FIG. 1 is a schematic block diagram of an endoscope system according to a first embodiment;
FIG. 2 is a side view of an operating section seen from a bending operating section side;
FIG. 3A is a perspective view showing a coupling configuration of a proximal end portion of the bending section and a distal end portion of a flexible tube section;
FIG. 3B is a cross-sectional view showing the coupling configuration of the proximal end portion of the bending section and the distal end portion of the flexible tube section;
FIG. 3C is a cross-sectional view taken along a line 3C-3C depicted in FIG. 3B;
FIG. 4A is a perspective view of a spiral rotary member in the first embodiment;
FIG. 4B is a cross-sectional view of the spiral rotary member depicted in FIG. 4A;
FIG. 4C is a view for explaining a function of a diameter change preventing section in a state where the spiral rotary member depicted in FIG. 4A is inserted in a lumen;
FIG. 5A is a perspective view of a spiral rotary member in a first modification of the first embodiment;
FIG. 5B is a cross-sectional view of the spiral rotary member depicted in FIG. 5A;
FIG. 6 is a cross-sectional view of a spiral rotary member in a second modification of the first embodiment.
FIG. 7A is a perspective view of a spiral rotary member in a second embodiment;
FIG. 7B is a cross-sectional view of the spiral rotary member depicted in FIG. 7A;
FIG. 7C is a view for explaining a function of a diameter change preventing section in a state where the spiral rotary member depicted in FIG. 7A is inserted in a lumen;
FIG. 8A is a perspective view of a spiral rotary member in a first modification of the second embodiment;
FIG. 8B is a cross-sectional view of the spiral rotary member depicted in FIG. 8A; and
FIG. 9 is a cross-sectional view of a spiral rotary member in a second modification of the second embodiment.

### Brief Description of Embodiments

Embodiments will now be described hereinafter in detail with reference to the drawings.

### [First Embodiment]

### [Configuration]

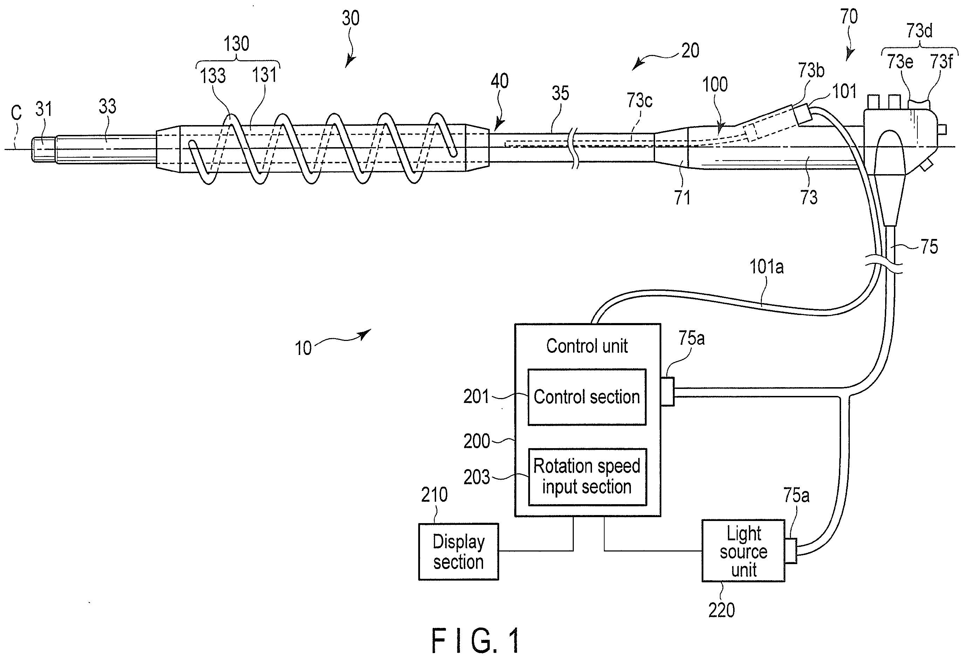

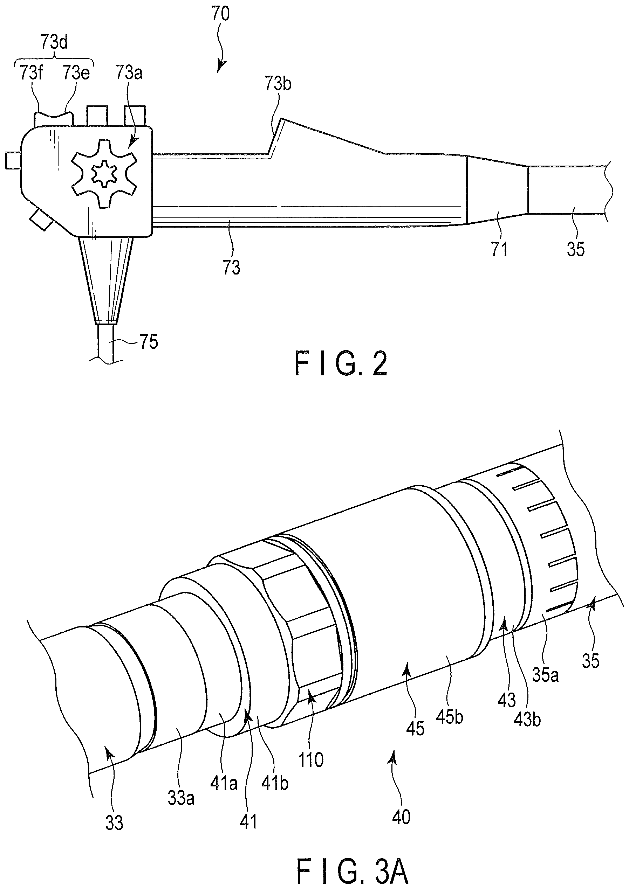

In reference to FIG. 1, FIG. 2, FIG. 3A, FIG. 3B, FIG. 3C, FIG. 4A, FIG. 4B, and FIG. 4C, a first embodiment will now be described. It is to be noted that some of members are omitted in some of the drawings to clarify the drawings, such as an example where a drive member 101 and a cable 101a are omitted in FIG. 2. Furthermore, in the following description, a longitudinal axis means a longitudinal axis C of an inserting section 30. A longitudinal axis direction means, e.g., a longitudinal axis direction of the inserting section 30. A radial direction means a radial direction of the inserting section 30.

### [Endoscope System 10]

As shown in FIG. 1, an endoscope system 10 has an endoscope 20 having an inserting section 30 that is inserted into or removed from, e.g., a lumen of a subject and a control unit 200 that controls a propulsive force that aids insertion and removal when the inserting section 30 is inserted into or removed from a lumen. The lumen means, e.g., a pylorus, a duodenum, a cardiac orifice, or the like.

Furthermore, as shown in FIG. 1, the endoscope system 10 also has a display section 210 that displays an image acquired by the endoscope 20 and a light source unit 220 arranged to emit light from a distal end portion of the inserting section 30 to an observation target. The image acquired by the endoscope 20 shows an observation target in a lumen. The observation target is, e.g., an affected part or a lesioned part in the lumen.

### [Configuration 1 of Endoscope 20]

As shown in FIG. 1, the endoscope 20 has the elongated inserting section 30 that is inserted into or removed from the lumen and has the longitudinal axis C and an operating section 70 that is coupled with a proximal end portion of the inserting section 30 and operates the endoscope 20. Such an endoscope 20 can be cleaned and sterilized.

### [Inserting Section 30]

As shown in FIG. 1, the inserting section 30 has a distal end rigid section 31, a bending section 33, and a flexible tube section 35 from a distal end portion side of the inserting section 30 toward a proximal end portion side of the inserting section 30. A proximal end portion of the distal end rigid section 31 is coupled with a distal end portion of the bending section 33, and a proximal end portion of the bending section 33 is coupled with a distal end portion of the flexible tube section 35.

The distal end rigid section 31 is the distal end portion of the inserting section 30, and it is rigid and does not bend. The distal end rigid section 31 has a imaging unit (not illustrated) that images an observation target and an emit section (not illustrated) from which light emits toward the observation target. That emit section is optically connected with the light source unit 220 and allows light guided from the light source unit 220 to exit toward the observation target.

The bending section 33 bends in desired directions, e.g., in up and down directions by an operation of a later-described bending operating section 73a shown in FIG. 2. When the bending section 33 bends, a position and a direction of the distal end rigid section 31 changes. As a result, the observation target (not illustrated) is illuminated with light, and the observation target is captured in an observation viewing field.

The flexible tube section 35 has desirable flexibility. Therefore, the flexible section 35 bends by external force. The flexible tube section functions as a tubular member extended from a later-described main body section 71 in the operating section 70. The flexible tube section 35 has, for example, a spiral tube section, a reticular tube section that is arranged on an outer side of this spiral tube section and covers the spiral tube section, and an outer tube that is arranged on an outer side of this reticular tube section and covers the reticular tube section. The reticular tube section is made of, for example, a metal, and the outer tube is made of, for example, a resin.

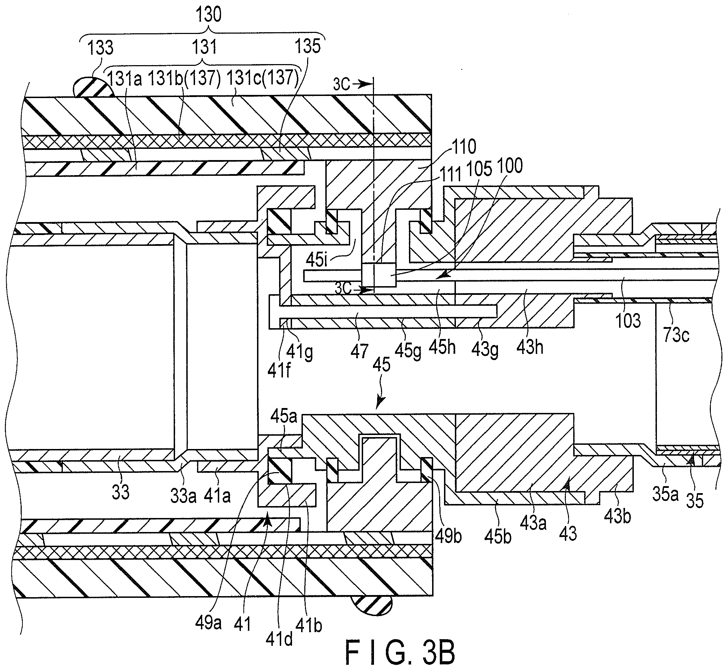

As shown in FIG. 3A and FIG. 3B, the proximal end portion of the bending section 33 is coupled with a bending section side mouth ring 33a. Moreover, as shown in FIG. 3A and FIG. 3B, the distal end portion of the flexible tube section 35 is coupled with a flexible tube section side mouth ring 35a.

### [Coupling Structure 40 of Proximal End Portion of Bending Section 33 and Distal End Portion of Flexible Tube section 35]

As shown in FIG. 3A and FIG. 3B, a coupling structure 40 has a cylindrical mouth ring 41 that fits to a proximal end portion of the bending section side mouth ring 33a to assure water-tightness and a cylindrical mouth ring 43 that fits to the flexible tube section side mouth ring 35a to assure water-tightness. Additionally, the coupling structure 40 further has a cylindrical member 45 coupled with the mouth ring 41 and the mouth ring 43 in the longitudinal axis direction to assure water-tightness and a coupling member 47 that couples the mouth ring 41, the mouth ring 43, and the cylindrical member 45 with each other. The coupling structure 40 can be cleaned and sterilized. The coupling member 47 has, for example, a pin or the like.

### [Mouth Ring 41]

As shown in FIG. 3B, the mouth ring 41 has a distal end portion 41a into which the bending section side mouth ring 33a is inserted and fitted when the mouth ring 41 is coupled with the bending section side mouth ring 33a and a proximal end portion 41b.

Furthermore, the mouth ring 41 also has an annular groove portion 41d into which a distal end portion 45a of the cylindrical member 45 is inserted to be fitted when the mouth ring 41 is coupled with the cylindrical member 45. The groove portion 41d is arranged at an edge portion of the proximal end portion 41b and concaved from the proximal end portion 41b toward the distal end portion 41a along the longitudinal axis direction.

As shown in FIG. 3B, the distal end portion 45a of the cylindrical member 45 is inserted into the groove portion 41d. As shown in FIG. 3B, in this state, a water-tightness assuring member 49a such as an O-ring is arranged in this groove portion 41d. The water-tightness assuring member 49a is in tight contact with the proximal end portion 41b of the mouth ring 41 and the distal end portion 45a of the cylindrical member 45 to assure water-tightness between the mouth ring 41 and the cylindrical member 45.

As shown in FIG. 3B, the mouth ring 41 has a tabular protruding portion 41f that is formed when part of a peripheral surface of the proximal end portion 41b protrudes toward the inner side of the mouth ring 41. The protruding portion 41f has an engagement hole portion 41g that pierces through the protruding portion 41f in the longitudinal axis direction of the mouth ring 41. The coupling member 47 pierces through and engages with the engagement hole portion 41g when the proximal end portion of the bending section 33 and the distal end portion of the flexible tube section 35 are coupled with.

### [Mouth Ring 43]

As shown in FIG. 3B, the mouth ring 43 has a distal end portion 43a that is inserted into and fitted to the proximal end portion 45b of the cylindrical member 45, and a proximal end portion 43b into which the flexible tube section side mouth ring 35a is inserted and fitted.

Additionally, as shown in FIG. 3B, the mouth ring 43 has a concave portion 43g that is arranged in the distal end portion 43a side and arranged on the same straight line as the engagement hole portion 41g in the longitudinal axis direction to engage with the coupling member 47 when the proximal end portion of the bending section 33 and the distal end portion of the flexible tube section 35 are coupled with.

As shown in FIG. 3B, the mouth ring 43 has a through-hole portion 43h that is arranged along the longitudinal axis direction of the mouth ring 43 and pierces through a thick wall portion of the mouth ring 43 and into which a later-described shaft member 103 is inserted. The through-hole portion 43h is arranged to deviate from a concave portion 43g in the radial direction of the mouth ring 43. The through-hole portion 43h is different from the concave portion 43g.

### [Cylindrical member 45]

As shown in FIG. 3B, the cylindrical member 45 has the distal end portion 45a that fits into the groove portion 41d of the mouth ring 41, and a proximal end portion 45b that fits to a distal end portion 43a of the mouth ring 43 while covering the distal end portion 43a of the mouth ring 43.

Furthermore, as shown in FIG. 3B, the cylindrical member 45 has a holding-hole portion 45g that holds the coupling member 47. The holding-hole portion 45g is arranged in a thick wall portion of the cylindrical member 45 and pierces through the thick wall portion of the cylindrical member 45 in the longitudinal axis direction of the cylindrical member 45. The holding-hole portion 45g is arranged on the same straight line with respect to an engagement hole portion 41g and the concave portion 43g in the longitudinal axis direction and communicates with the engagement hole portion 41g and the concave portion 43g when the proximal end portion of the bending section 33 and the distal end portion of the flexible tube section 35 are coupled with. The holding-hole portion 45g holds the coupling member 47 when the coupling member 47 pierces through the holding-hole portion 45g.

Moreover, the cylindrical member 45 also has a gear arrangement section 45h where a distal end portion of the shaft member 103 and a later-described gear member 105 are arranged. The gear arrangement section 45h is formed as a hollow portion in which the distal end portion of the shaft member 103 and the gear member 105 are arranged. The gear arrangement section 45h is arranged in the thick wall portion of the cylindrical member 45 in such a manner that the gear arrangement section 45h communicates with the through-hole portion 43h when the proximal end portion of the bending section 33 and the distal end portion of the flexible tube section 35 are coupled with. The gear arrangement section 45h is arranged to be offset from the holding-hole portion 45g in the radial direction of the cylindrical member 45. The gear arrangement section 45h is different from the holding-hole portion 45g.

Additionally, the cylindrical member 45 has an opening portion 45i that is arranged in part of a peripheral surface of the cylindrical member 45 and communicates with the outside and the gear arrangement section 45h in the radial direction of the cylindrical member 45.

### [Example of Coupling of Proximal End Portion of Bending Section 33 and Distal End Portion of Flexible Tube Section 35]

### (Step 1)

The flexible tube section side mouth ring 35a is inserted into and fitted to the proximal end portion 43b of the mouth ring 43.

### (Step 2)

Then, the distal end portion 43a of the mouth ring 43 is inserted into the proximal end portion 45b of the cylindrical member 45 so that the gear arrangement section 45h is arranged on the same straight line as the through-hole portion 43h in the longitudinal axis direction and communicates with the through-hole portion 43h, and so that the concave portion 43g is arranged on the same straight line as the holding-hole portion 45g in the longitudinal axis direction and communicates with the holding-hole portion 45g. At this time, the distal end portion 43a of the mouth ring 43 is covered with the proximal end portion 45b of the cylindrical member 45 and fits into the proximal end portion 45b of the cylindrical member 45.

When the mouth ring 43 is fitted to the cylindrical member 45, the mouth ring 43 and the cylindrical member 45 assure water-tightness with each other.

### (Step 3)

The water-tightness assuring member 49a is arranged in the groove portion 41d.

Then, the distal end portion 45a of the cylindrical member 45 is inserted and fitted into the groove portion 41d so that the engagement hole portion 41g, the concave portion 43g, and the holding-hole portion 45g are arranged on the same straight line in the longitudinal axis direction each other, and so that the engagement hole portion 41g communicates with the holding-hole portion 45g.

At this time, the water-tightness assuring member 49a is in tight contact with the proximal end portion 41b of the mouth ring 41 and the distal end portion 45a of the cylindrical member 45 to assure water-tightness between the mouth ring 41 and the cylindrical member 45.

### (Step 4)

Subsequently, the coupling member 47 pierces through the engagement hole portion 41g and the holding-hole portion 45g and engages with the concave portion 43g. As a result, the flexible tube section 35, the mouth ring 43, the cylindrical member 45, and the mouth ring 41 are coupled with.

### (Step 5)

Furthermore, the bending section side mouth ring 33a is inserted and fitted into the distal end portion 41a of the mouth ring 41. As a result, the proximal end portion of the bending section 33 and the distal end portion of the flexible tube section 35 are coupled with.

### [Operating Section 70]

As shown in FIG. 1, the operating section 70 has the main body section 71 from which the flexible tube section 35 is extended, a grip section 73 that is coupled with the proximal end portion of the main body section 71 and gripped by an operator who operates the endoscope 20, and a universal cord 75 connected with the grip section 73.

As shown in FIG. 1 and FIG. 2, the grip section 73 has the bending operating section 73a that operates to bend the bending section 33, a drive member insertion opening 73b into which a later-described drive member 101 is inserted, and a rotary operating section 73d that operates a rotating direction of the later-described shaft member 103.

The bending operating section 73a is connected with a non-illustrated operation wire that is inserted into an inner side of the grip section 73, the main body section 71, and the flexible tube section 35. A distal end portion of the operation wire is coupled with the distal end section of the bending section 33. When the bending operating section 73a is operated, the operation wire is pulled. When the operation wire is pulled, the bending section 33 bends.

As shown in FIG. 1, the drive member insertion opening 73b is coupled with a proximal end portion of a shaft member insertion channel 73c. The drive member insertion opening 73b is an insertion opening through which the shaft member 103 is inserted into the shaft member insertion channel 73c. As shown in FIG. 1 and FIG. 3B, the shaft member insertion channel 73c is arranged to extend from the grip section 73 to the flexible tube section 35 via the main body section 71 an inner side of the inserting section 30. Furthermore, a distal end portion of the shaft member insertion channel 73c is coupled with the mouth ring 43 to communicate with the through-hole portion 43h.

As shown in FIG. 1 and FIG. 2, the rotary operating section 73d has a counterclockwise operating section 73e that operates the shaft member 103 so that the shaft member 103 rotates in a counterclockwise direction by drive force of the drive member 101 and a clockwise operating section 73f that operates the shaft member 103 so that the shaft member 103 rotates in a clockwise direction by the drive force of the drive member 101. The counterclockwise operating section 73e and the clockwise operating section 73f are connected with the control unit 200 through the universal cord 75 and a connecting section 75a.

As shown in FIG. 1, the universal cord 75 has the connecting sections 75a that is connected to the control unit 200 and the light source unit 220.

### [Configuration 2 of Endoscope 20]

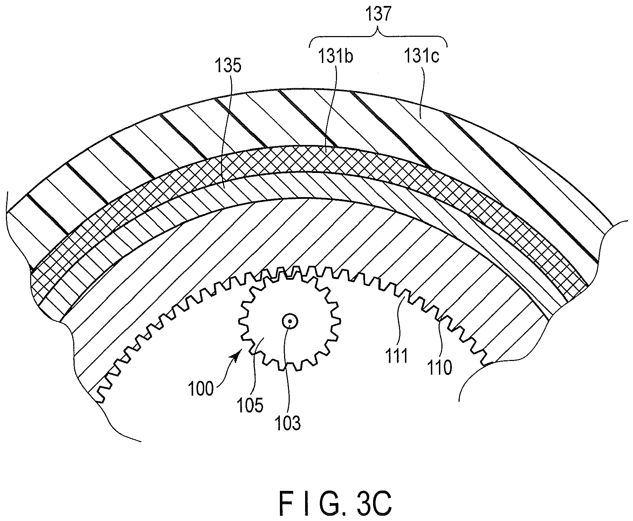

As shown in FIG. 1, FIG. 3B, and FIG. 3C, the endoscope 20 also has a rotary drive mechanism 100, a first rotary member 110, and a spiral rotary member 130. The rotary drive mechanism 100, the first rotary member 110, and the spiral rotary member 130 function as an auxiliary propulsion unit which imparts to the inserting section 30 the propulsive force to enable insertion of the inserting section 30 into a lumen or removal therefrom, thereby aiding propulsion of the inserting section 30. The propulsive force means an insertion force that acts on the inserting section 30 in the insertion direction of the inserting section 30 to aid the insertion of the inserting section 30, or a removal force that acts on the inserting section 30 in the removal direction of the inserting section 30 to aid the removal of the inserting section 30.

For example, the rotary drive mechanism 100, the first rotary member 110, and the spiral rotary member 130 function as an auxiliary insertion and removal unit that aids insertion and removal of the inserting section 30 into or from a lumen.

### [Rotary Drive Mechanism 100]

As shown in FIG. 1, FIG. 3B, and FIG. 3C, the rotary drive mechanism 100 is arranged on an inner side of the inserting section 30. As shown in FIG. 1, FIG. 3B, and FIG. 3C, the rotary drive mechanism 100 has the drive member 101 that is connected with the control unit 200 through the cable 101a and inserted into the drive member insertion opening 73b, the shaft member 103 that rotates in a periaxial direction of the longitudinal axis of the first rotary member 110 by drive force of the drive member 101, and a gear member 105 that is arranged at the distal end portion of the shaft member 103 and functions as an outer peripheral teeth section. The shaft member 103 has a distal end portion and a proximal end portion coupled with the drive member 101.

The drive member 101 has, e.g., a motor or the like. The drive member 101 has drive force that enables the first rotary member 110 to rotate in the periaxial direction of the longitudinal axis of the first rotary member 110.

As shown in FIG. 3B, the shaft member 103 is inserted into the shaft member insertion channel 73c. The shaft member 103 is arranged along the longitudinal axis direction. The shaft member 103 has, e.g., a flexible torque wire. The shaft member 103 rotates in the periaxial direction of the longitudinal axis of the shaft member 103 by the drive force of the drive member 101. The distal end portion of the shaft member 103 is arranged in the gear arrangement section 45h as shown in FIG. 3B.

As shown in FIG. 3B and FIG. 3C, the gear member 105 is arranged in the gear arrangement section 45h to mesh with a later-described inner peripheral teeth section 111 of the first rotary member 110. The gear member 105 is arranged at the distal end portion of the shaft member 103 so that it rotates in the periaxial direction of the gear member 105 in accordance with rotation of the shaft member 103. Furthermore, the gear member 105 rotates in the periaxial direction of the gear member 105 in accordance with rotation of the shaft member 103 in a state where the gear member 105 meshes with the first rotary member 110, thereby rotating the first rotary member 110.

The shaft member 103 and the gear member 105 are transmission rotary members that transmit the drive force of the drive member 101 to the first rotary member 110 and rotates the first rotary member 110 by the drive force.

### [First Rotary Member 110]

As shown in FIG. 3B, the first rotary member 110 has, for example, a gear base member. The first rotary member 110 has, for example, a cylindrical shape. As shown in FIG. 3B and FIG. 3C, the first rotary member 110 has an inner peripheral teeth section 111 that is arranged on an inner peripheral surface of the first rotary member 110 and meshes with the gear member 105. This inner peripheral teeth section 111 has a ring shape.

As shown in FIG. 3C, when the gear member 105 rotates around the axis of the gear member 105 in a state where the gear member 105 meshes with the inner peripheral teeth section 111, the first rotary member 110 rotates together with the inner peripheral teeth section 111 around the axis of the first rotary member 110. As described above, the first rotary member 110 is coupled with the rotary drive mechanism 100 and rotates around the axis of the first rotary member 110 by the rotary drive mechanism 100.

As shown in FIG. 3B and FIG. 3C, the first rotary member 110 is arranged in such a manner that, for example, the inner peripheral teeth section 111 is inserted into the opening portion 45i and arranged in the gear arrangement section 45h, the inner peripheral teeth section 111 meshes with the gear member 105 arranged in the gear arrangement section 45h, and an outer peripheral surface of the first rotary member 110 outwardly protrudes in the radial direction of the cylindrical member 45 beyond outer peripheral surfaces of the mouth rings 41 and 43 and an outer peripheral surface of the cylindrical member 45.

Furthermore, the first rotary member 110 is arranged to assure water-tightness with the cylindrical member 45. Therefore, as shown in FIG. 3B, a water-tightness assuring member 49b such as an O-ring is arranged between the first rotary member 110 and the cylindrical member 45 in the radial direction of the cylindrical member 45.

### [Spiral Rotary Member 130]

As shown in FIG. 1, FIG. 3B, FIG. 4A, and FIG. 4B, the spiral rotary member 130 functions as an auxiliary insertion and removal device that is inserted into or removed from a lumen in a state where the inserting section 30 having the longitudinal axis C is inserted into, thereby aiding insertion and removal of the inserting section 30 into or from the lumen.

As shown in FIG. 1, FIG. 3B, FIG. 4A, and FIG. 4B, the spiral rotary member 130 has a tubular main body section 131 into which the inserting section 30 can be inserted and can rotate around the longitudinal axis C, and a fin section 133 that is arranged on an outer peripheral surface of the main body section 131 and spirally arranged in a desired direction to be wound around the longitudinal axis C. The desired direction will be described later. The main body section 131 may have a tubular shape, and it may have, for example, a cylindrical shape or a square tubular shape.

Additionally, as shown in FIG. 1, FIG. 3B, FIG. 4A, and FIG. 4B, the spiral rotary member 130 also has a spiral tube section 135 that is arranged on the main body section 131 and spirally arranged to be wound around the longitudinal axis C in the same direction as the fin section 133 and a diameter change preventing section 137 that prevents a diameter of the spiral tube section 135 from expanding.

### [Main Body Section 131]

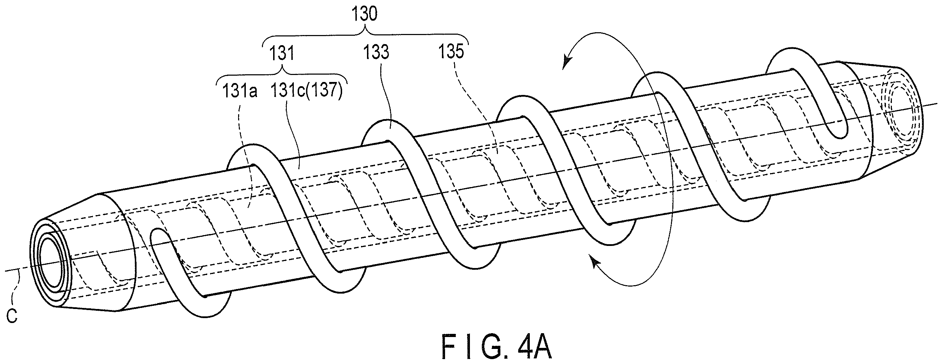

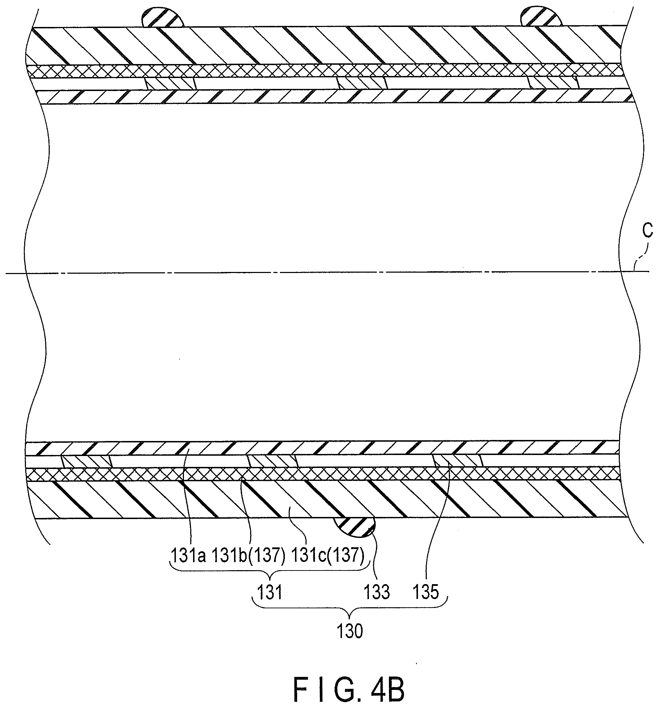

As shown in FIG. 3B, FIG. 4A, and FIG. 4B, the main body section 131 has, e.g., an inner tube 131a that is arranged on an inner side of the spiral tube section 135 and covered by the spiral tube section 135, a reticular tube section 131b that is arranged on an outer side of the spiral tube section 135 and covers the spiral tube section 135, and an outer tube 131c that is arranged on an outer side of the reticular tube section 131b and covers the reticular tube section 131b. The reticular tube section 131b has a tubular shape. The reticular tube section 131b may have a tubular shape, and it may have, for example, a cylindrical shape or a square tubular shape. An outer surface of the reticular tube section 131b is formed into a reticular pattern. It is to be noted that the reticular tube section 131b is omitted in FIG. 4A to clarify the drawing.

As shown in FIG. 3B, FIG. 4A, and FIG. 4B, the inner tube 131a is formed into a substantially circular tube shape so that the inserting section 30 can be inserted in the inner tube 131a. The inner tube 131a functions as a prevention material that prevents the inserting section 30 and the spiral tube section 135 from abrading each other. The inner tube 131a is made of a resin material having flexibility such as a rubber material. The inner tube 131a is made of, for example, a cleanable and sterilizable resin material. Such a resin material is, e.g., polyurethane. That is, the inner tube 131a functions as an inner resin layer. Furthermore, the inner tube 131a is thinner than the outer tube 131c and functions as an inner thin wall portion of the main body section 131.

As shown in FIG. 3B, FIG. 4A, and FIG. 4B, the outer tube 131c is formed into a substantially circular tube shape to cover the outer side of the reticular tube section 131b. The outer tube 131c is made of a resin material having flexibility such as a rubber material. The outer tube 131c is made of, for example, a cleanable and sterilizable resin material. Such a resin material is, e.g., polyurethane. That is, the outer tube 131c functions as an outer resin layer. The outer tube 131c is thicker than the inner tube 131a and functions as an outer thick wall portion of the main body section 131. The outer tube 131c has substantially the same length as the spiral tube section 135 together with reticular tube section 131b so that the outer tube 131c can cover the entire spiral tube section 135 together with reticular tube section 131b.

As shown in FIG. 3B and FIG. 4B, in this embodiment, the inner tube 131a and the outer tube 131c sandwich the spiral tube section 135 therebetween through the reticular tube section 131b in the radial direction of the main body section 131. That is, the main body section 131 holds the spiral tube section 135 so that the spiral tube section 135 is imbedded in the main body section 131.

In the main body section 131, the outer peripheral surface of the inner tube 131a is bonded to the inner peripheral surface of the spiral tube section 135 by, for example, an adhesive (not illustrated). Furthermore, a distal end portion of the inner tube 131a is bonded to a distal end portion of the spiral tube section 135, and a proximal end portion of the inner tube 131a is bonded to a proximal end portion side of the spiral tube section 135 as shown in FIG. 3B.

Moreover, in the main body section 131, the entire inner peripheral surface of the outer tube 131c is bonded to the entire outer peripheral surface of the spiral tube section 135 by, for example, the non-illustrated adhesive through the reticular tube section 131b. Additionally, a distal end portion of the outer tube 131c is bonded to the distal end portion of the spiral tube section 135, and a proximal end portion of the outer tube 131c is bonded to the proximal end portion of the spiral tube section 135.

It is to be noted that, in view of the above description, the outer tube 131c is bonded to the inner tube 131a by, e.g., the adhesive through a gap of the reticular tube section 131b and a gap of the spiral tube section 135. Furthermore, the main body section 131 is integrated with the spiral tube section 135. Therefore, although the details will be described later, when the spiral tube section 135 rotates around the longitudinal axis C, the main body section 131 rotates around the longitudinal axis C in the same direction as the spiral tube section 135.

Furthermore, in this embodiment, the main body section 131 can suffice if it has at least the outer tube 131c alone. If the main body section 131 has the outer tube 131c alone, the outer tube 131c is directly bonded to the outer peripheral surface of the spiral tube section 135 by the adhesive, for example.

Moreover, the inner tube 131a does not have to be bonded to the spiral tube section 135. In this case, the main body section 131 has the outer tube 131c and the reticular tube section 131b, and the main body section 131 is different from the inner tube 131a.

### [Fin Section 133]

As shown in FIG. 1, FIG. 3B, FIG. 4A, and FIG. 4B, the fin section 133 is made of a cleanable and sterilizable resin such as rubber. The fin section 133 is fixed on the outer peripheral surface of the outer tube 131c by adhesion or welding, for example. As shown in FIG. 1 and FIG. 4A, the fin section 133 is arranged to be spiral in, for example, a clockwise fashion in a direction along which the distal end portion of the main body section 131 is seen from the proximal end portion of the main body section 131. The desired direction includes this clockwise fashion. The fin section 133 is erected on the outer tube 131c.

For example, when the inserting section 30 is inserted into a lumen, the fin section 133 abuts on an inner wall of the lumen. In this state, when the main body section 131 rotates around the longitudinal axis C, the fin section 133 engages with the inner wall of the lumen, and the propulsive force acts on the inserting section 30 in the longitudinal axis direction. As a result, the inserting section 30 moves forward and backward in the lumen.

When the main body section 131 rotates in the clockwise direction, the insertion force acts on the inserting section 30, and insertability of the inserting section 30 is improved. Moreover, when the main body section 131 rotates in the counterclockwise direction, the removal force acts on the inserting section 30, and removability of the inserting section 30 is improved.

### [Spiral Tube Section 135]

As shown in FIG. 1, FIG. 3B, FIG. 4A, and FIG. 4B, the spiral tube section 135 is arranged to be wound around the longitudinal axis C in the same direction as the fin section 133, i.e., the clockwise direction. As described above, the spiral tube section 135 is spirally arranged in the clockwise fashion like the fin section 133 in the direction along which the distal end portion of the main body section 131 is seen from the proximal end portion of the main body section 131. As shown in FIG. 4A, for example, the spiral tube section 135 is more closely wound than the fin section 133. That is, the number of wounds of the spiral tube section 135 is higher than, e.g., the number of wounds of the fin section 133.

The spiral tube section 135 is formed into a substantially circular tube shape by forming, for example, a strip-like thin plate material into a spiral shape. The thin plate material is, for example, stainless steel material. Each of the distal end portion of the spiral tube section 135 and the proximal end portion of the spiral tube section 135 is cut to form substantially a 90 degree angle with respect to the central axis of the spiral tube section 135. The spiral tube section 135 is, for example, a thin-wall metal spiral tube. The spiral tube section 135 is arranged over the entire main body section 131 along the longitudinal (axis) direction of the main body section 131 to prevent collapse of the entire main body section 131 and local collapse of the main body section 131. The spiral tube section 135 has a thickness that is uniform from the distal end portion of the spiral tube section 135 to the proximal end portion of the spiral tube section 135.

As shown in FIG. 3B and FIG. 4B, the spiral tube section 135 is sandwiched between the inner tube 131a and the reticular tube section 131b in the radial direction of the spiral tube section 135 and bonded to the inner tube 131a and the reticular tube section 131b as described above.

As shown in FIG. 3B, the proximal end portion of the spiral tube section 135 is disposed to the outer peripheral surface of the first rotary member 110. Therefore, when the first rotary member 110 rotates around the axis of the first rotary member 110, the spiral tube section 135 rotates around the longitudinal axis C. Each of the first rotary member 110 and the spiral tube section 135 is also an attachment section that attaches the spiral rotary member 130 to the inserting section 30. Furthermore, the spiral rotary member 130 including the spiral tube section 135 is detachably attached to the inserting section 30 in such a manner that the inserting section 30 is inserted into the spiral rotary member 130 and the spiral rotary member 130 can rotate around the longitudinal axis C with respect to the inserting section 30.

### [Diameter Change Preventing Section 137]

As shown in FIG. 3B, FIG. 4A, and FIG. 4B, in a case where the spiral tube section 135 is wound in the same direction as the fin section 133 as described in this embodiment, the diameter change preventing section 137 is arranged on the outer side than the spiral tube section 135. In this embodiment, for example, the diameter change preventing section 137 has the outer tube 131c that is arranged in the main body section 131 and functions as the outer thick wall portion of the main body section 131, and has the reticular tube section 131b that is arranged in the main body section 131 and arranged between the spiral tube section 135 and the outer tube 131c in the radial direction of the spiral rotary member 130. In other words, each of the reticular tube section 131b and the outer tube 131c also functions as the diameter change preventing section 137. It is to be noted that the diameter change preventing section 137 having at least the outer tube 131c can suffice.

In general, when the fin section 133 is wound in the clockwise direction and the main body section 131 rotates in the winding direction of the fin section 133, i.e., the clockwise direction, the fin section 133 pulls in the inner wall of the lumen. As a result, the inserting section 30 moves forward in the lumen. Furthermore, in a symmetrical fashion, when the main body section 131 rotates in the opposite direction of the winding direction of the fin section 133, e.g., the counterclockwise direction, the fin section 133 opens the inner walls of the lumen. As a result, the inserting section 30 moves backward in the lumen.

Under these circumstances, a first rotating force that rotates the main body section 131 in the clockwise direction is higher than a second rotating force that rotates the main body section 131 in the counterclockwise direction. The first rotating force advances the inserting section 30, and the second rotating force retreats the inserting section 30. That is, torque at the time of advancement (insertion) is higher than torque at the time of retreat (removal).

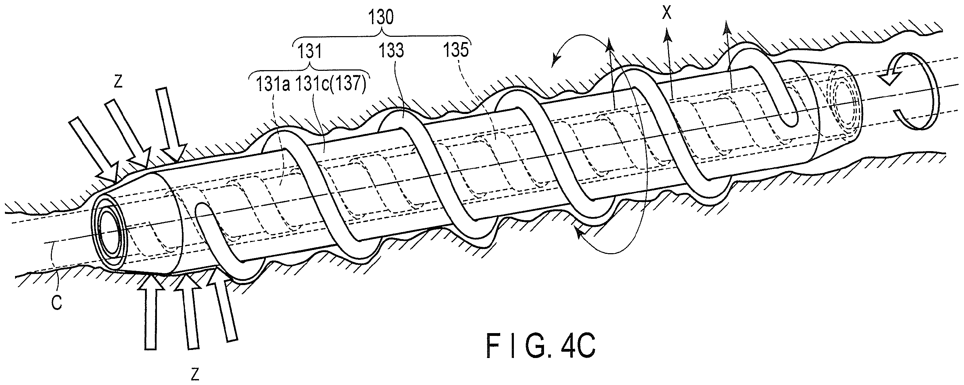

In this embodiment, when the spiral tube section 135 wound in the clockwise direction rotates in the clockwise direction, a force that opens up the spiral is applied to the spiral tube section 135. Therefore, the diameter of the spiral tube section 135 is to expand in, for example, the X direction as shown in FIG. 4C. However, the outer peripheral surface of the spiral tube section 135 is bonded to the entire reticular tube section 131 and also bonded to the entire outer tube 131c through the reticular tube section 131b. Therefore, the diameter change preventing section 137 including the reticular tube section 131b and the outer tube 131c presses the spiral tube section 135 against the diameter expansion. Therefore, in FIG. 4C showing the state where the spiral rotary member 130 is inserted in the lumen, the diameter change preventing section 137 including the reticular tube section 131b and the outer tube 131c avoids the diameter expansion with respect to the spiral tube section 135 that is to radially expand in the X direction as shown in FIG. 4C. In more detail, the diameter change preventing section 137 applies a counter force in the opposite direction of the X direction shown in FIG. 4C that prevents the diameter expansion of the spiral tube section 135, thereby preventing the diameter of the spiral tube section 135 from expanding. As a result, when the spiral rotary member 130 rotates in the clockwise direction, the rotating force of the spiral tube section 135 is efficiently transmitted to the entire reticular tube section 131b and the entire outer tube 131c that function as the diameter change preventing section 137. Moreover, when the outer tube 131c rotates in the clockwise direction, the fin section 133 also rotates in the clockwise direction, and the fin section 133 pulls in the inner wall of the lumen. Therefore, the inserting section 30 moves forward in the lumen.

As described above, the first rotating force is not transmitted to part of the main body section 131, that is to say the proximal end portion of the outer tube 131c from the first rotation member 110. When the spiral tube section 135 is arranged, the first rotating force is transmitted from the first rotation member 110 to the entire main body section 131, in other words, the entire outer tube 131c through the spiral tube section 135. As a result, for example, as shown in FIG. 4C, on the distal end side of the spiral rotary member 130, even if the fin section 133 and the main body section 131 receive resistance Z from the inner wall of the lumen, the rotating force applied to the spiral rotary member 130 is efficiently transmitted to the whole from the proximal end side toward the distal end side of the spiral rotary member 130. Therefore, rotation of the spiral rotary member 130 is prevented from being stopped by the resistance.

### [Control Unit 200]

As shown in FIG. 1, the control unit 200 has a control section 201 that controls the drive of the drive member 101, the display section 210, and the light source unit 220, and has a rotation speed input section 203 that minutely inputs a rotation speed of the drive member 101, in more detail the spiral rotation member 130.

The control section 201 controls a rotating direction of the drive member 101 in accordance with an operation of the counterclockwise operating section 73e or the clockwise operating section 73f. Additionally, the control section 201 controls a rotation speed of the drive member 101 and controls a rotation speed of the spiral rotary member 130 based on an input amount of the rotation speed input section 203.

### [Operation]

When the clockwise operating section 73f and the rotation speed input section 203 are operated, the control section 201 controls the rotating direction of the drive member 101 so that the drive member 101 rotates in the clockwise direction, and controls the rotation speed of the drive member 101 based on the input amount of the rotation speed input section 203.

The shaft member 103 coupled with the drive member 101 and the gear member 105 arranged at the distal end portion of the shaft member 103 rotate around the longitudinal axis C in the clockwise direction. As a result, the first rotation member 110 having the inner peripheral teeth section 111 that meshes with the gear member 105 and the spiral tube section 135 attached to the first rotary member 110 rotate around the longitudinal axis C in the clockwise direction.

When the spiral tube section 135 rotates in the clockwise direction, the diameter of the spiral tube section 135 is to expand, but the diameter change preventing section 137 prevents the diameter of the spiral tube section 135 from expanding. As a result, when the spiral rotary member 130 rotates in the clockwise direction, the rotating force of the spiral tube section 135 is efficiently transmitted to the entire outer tube 131c that functions as the diameter change preventing section 137. Furthermore, when the outer tube 131c rotates in the clockwise direction, the fin section 133 also rotates in the clockwise direction, and the fin section 133 pulls in the inner wall of the lumen. Therefore, the inserting section 30 moves forward in the lumen.

As described above, the first rotating force is not transmitted to part of the main body section 131 like the proximal end portion of the outer tube 131 from the first rotary member 110. Since the spiral tube section 135 is arranged, the first rotating force is transmitted from a position where the first rotating force is transmitted to the end portion of the main body section 131 through the spiral tube section 135, the reticular tube section 131b, and the outer tube 131c Therefore, in comparison to a case where the first rotating force is transmitted to part of the main body section 131, transmissibility of the rotating force to the spiral rotary member 130 is improved in this embodiment. Therefore, even if the fin section 133 and the main body section 131 receive resistance from the inner wall of the lumen, rotation of the spiral rotary member 130 can be prevented from being stopped by the resistance, and the spiral rotary member 130 rotates. Therefore, the fin section 133 pull in the inner wall of the lumen, and the inserting section 30 moves forward in the lumen.

Furthermore, the outer tube 131c has substantially the same length as the spiral tube section 135 to cover the entire spiral tube section 135, and it is bonded to the entire spiral tube section 135. Therefore, the outer tube 131c prevents part of the spiral tube section 135 and the entire spiral tube section 135 from being deformed. That is, the first rotating force is efficiently transmitted to the entire main body section 131 (the outer tube 131c) from the spiral rotary section.

### [Effect]

In this embodiment, winding the fin section 133 and the spiral tube section 135 in the clockwise direction enables transmitting the first rotating force to the entire main body section 131, that is, the entire outer tube 131c through the spiral tube section 135 from the first rotary member 110. Therefore, in this embodiment, as compared to the case where the first rotating force is transmitted to part of the main body section 131, the transmissibility of the rotating force to the spiral rotary member 130 can be improved.

As a result, in this embodiment, even if the fin section 133 and the main body section 131 receive the resistance from the inner wall of the lumen, the rotation of the spiral rotary member 130 can be prevented from being stopped by the resistance, and the spiral rotary member 130 can be rotated. Moreover, in this embodiment, the fin section 133 can pull in the inner wall of the lumen, and the inserting section 30 can move forward in the lumen. That is, in this embodiment, the transmissibility of the rotating force at the time of insertion can be improved.

Additionally, in this embodiment, when the spiral tube section 135 and the fin section 133 are wound in the same direction each other, the diameter change preventing section 137 is arranged on the outer side than the spiral tube section 135. As a result, in this embodiment, the outer tube 131c that functions as the diameter change preventing section 137 can prevent the diameter of the spiral tube section 135 that rotates in, e.g., the clockwise direction from expanding. Therefore, in this embodiment, when the spiral rotary member 130 rotates in the clockwise direction, the rotating force of the spiral tube section 135 can be efficiently transmitted to the entire outer tube 131c that functions as the diameter change preventing section 137. In this embodiment, the fin section 133 can also rotate in the clockwise direction, and the inserting section 30 can move forward in the lumen.

In this embodiment, the main body section 131 holds the spiral tube section 135 so that the spiral tube section 135 is imbedded in the main body section 131. As a result, in this embodiment, the first rotating force can be efficiently transmitted to the main body section 131.

Moreover, in this embodiment, since the outer tube 131c is bonded to the entire spiral tube section 135, the first rotating force can be efficiently transmitted to the main body section 131.

Additionally, in this embodiment, the outer tube 131c that functions as the diameter change preventing section 137 is thicker than the inner tube 131a. As a result, in this embodiment, the outer tube 131c can avoid the diameter expansion of the spiral tube section 135, and the main body section 131 can be thinned in comparison to a case where the outer tube 131c and the inner tube 131a are sufficiently thick.

Also, in this embodiment, the inner tube 131a can prevent the inserting section 30 and the spiral tube section 135 from abrading each other.

### [Modification 1]

In the above-described embodiment, the description has been given for the example where the fin section 133 is wound in the clockwise direction and the spiral tube section 135 is likewise wound in the clockwise direction.

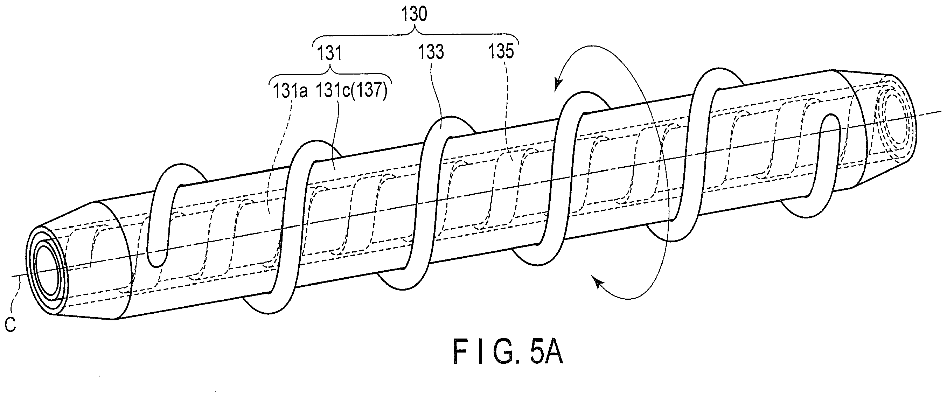

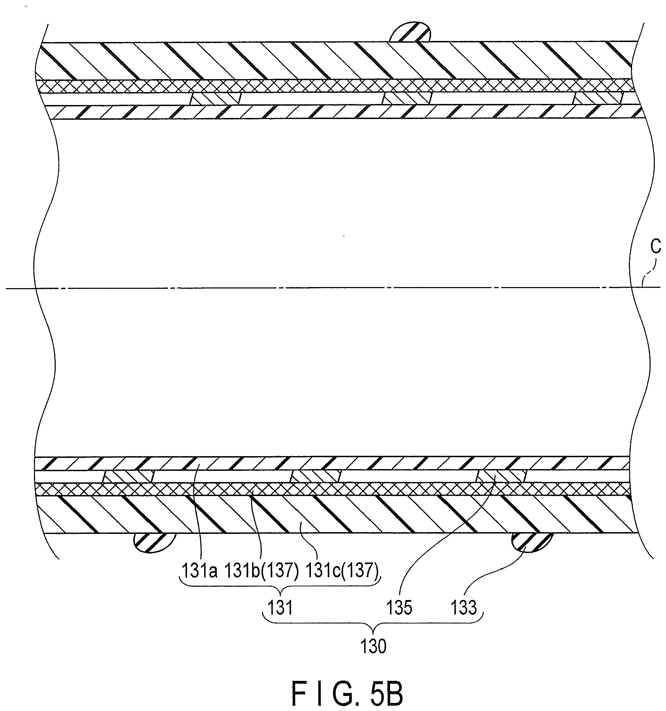

As shown in FIG. 5A and FIG. 5B and as in this modification, the fin section 133 may be spirally arranged in, for example, the counterclockwise fashion in a direction along which the distal end portion of the main body section 131 is seen from the proximal end portion of the same. Thus, in this case, the spiral tube section 135 can be arranged to be wound around the longitudinal axis C in the same direction as the fin section 133, i.e., the counterclockwise direction. As described above, the spiral tube section 135 is spirally arranged in the counterclockwise fashion like the fin section 133 in the direction along which the distal end portion of the main body section 133 is seen from the proximal end portion of the same. The desired direction includes this counterclockwise direction. Note that the reticular tube section 131b is omitted in FIG. 5A to clarify the drawing.

As described above, the spiral tube section 135 being wound in the same direction as the fin section 133 can suffice. Furthermore, in this case, the diameter change preventing section 137 has the outer tube 131c that functions as the outer thick wall portion of the main body section 131 arranged on the outer side than the spiral tube section 135, and the reticular tube section 131b arranged between the spiral tube section 135 and the outer tube 131c in the radial direction of the spiral rotary member 130. Likewise, the diameter change preventing section 137 having at least the outer tube 131c can suffice.

In this modification, winding the fin section 133 and the spiral tube section 135 in the counterclockwise direction enables transmitting the second rotating force from the first rotary member 110 to the entire main body section 131, e.g., the entire outer tube 131c through the spiral tube section 135. Therefore, in this modification, as compared to a case where the second rotating force is transmitted to part of the main body section 131, the transmissibility of the rotating force to the spiral rotary member 130 can be improved. Here, in this modification, since the fin section 133 is wound in the counterclockwise direction, the inserting section 30 moves forward in the lumen when the second rotating force is applied to the spiral rotary member 130, and the inserting section 30 moves backward in the lumen when the first rotating force is applied to the spiral rotary member 130.

As a result, in this modification, even if the fin section 133 and the main body section 131 receive resistance from the inner wall of the lumen, it is possible to prevent the resistance from stopping the rotation of the spiral rotary member 130, thereby rotating the spiral rotary member 130. Moreover, in this modification, the fin section 133 can pull in the inner wall of the lumen, and the inserting section 30 can move forward in the lumen. Additionally, the transmissibility of the rotating force at the time of removal can be improved.

Additionally, in this modification, the main body section 131 holds the spiral tube section 135 so that the spiral tube section 135 can be embedded in the main body section 131. As a result, in this modification, the second rotating force can be efficiently transmitted to the main body section 131.

Furthermore, in this modification, since the outer tube 131c is bonded to the entire spiral tube section 135, the second rotating force can be efficiently transmitted to the main body section 131.

### [Modification 2]

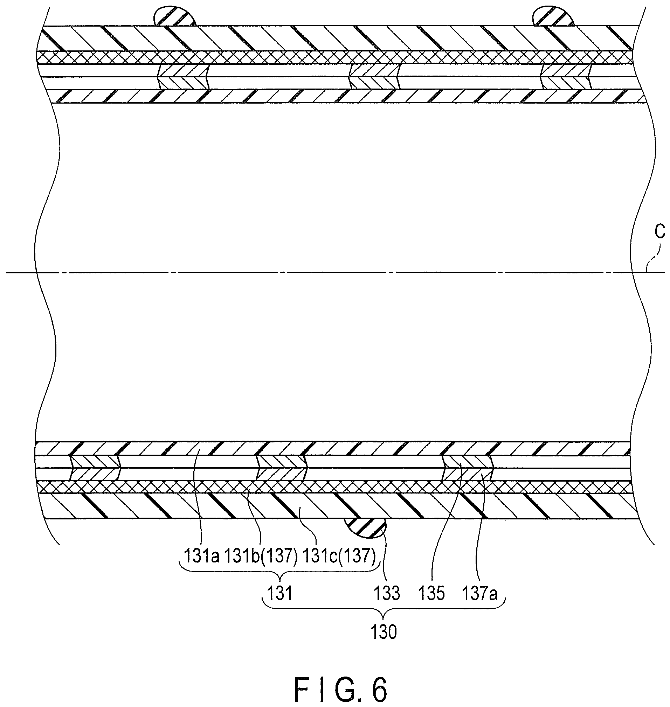

As shown in FIG. 6, the diameter change preventing section 137 also has a diameter change preventing spiral tube section (which will be referred to as a spiral tube section 137a hereinafter) arranged on the outer side of the spiral tube section 135 when the spiral tube section 135 is wound in the same direction as the fin section 133. The spiral tube section 137a is spirally arranged to be wound around the longitudinal axis C in the direction opposite to the spiral tube section 135. The spiral tube section 137a has substantially the same configuration as the spiral tube section 135.

The spiral tube section 137a is sandwiched between the spiral tube section 135 and the reticular tube section 131b in the radial direction of the spiral tube section 135. The spiral tube section 137a is bonded to the spiral tube section 135 and bonded to the outer tube 131c through the reticular tube section 131b.

When the configuration of this modification is combined with the configuration of the first embodiment, the spiral tube section 137a is wound in the counterclockwise direction as shown in FIG. 6. As described above, in a direction along which the distal end portion of the main body section 131 is seen from the proximal end portion of the same, the spiral tube section 137a is spirally arranged in the direction opposite to the fin section 133 and the spiral tube section 135, in other words, the counterclockwise direction.

Although not shown, when the configuration of this modification is combined with the configuration of the modification 1 according to the first embodiment (see FIG. 5A and FIG. 5B), the spiral tube section 137a is wound in the clockwise direction.

In this modification, when the spiral rotary member 130 is rotated in the clockwise direction, the diameter of the spiral tube section 137a is to contract. Therefore, the spiral tube section 137a can further prevent the diameter expansion of the spiral tube section 135 that rotates in, for example, the clockwise direction in the first embodiment. As a result, in this modification, when the spiral rotary member 130 rotates in the clockwise direction, the rotating force of the spiral tube section 135 can be efficiently transmitted to the entire outer tube 131c and the entire spiral tube section 137a that function as the diameter change preventing section 137. Moreover, in this modification, the fin section 133 can also rotate in the clockwise direction, and the inserting section 30 can move forward in the lumen. Additionally, in this modification, the fin section 133 can also rotate in the counterclockwise direction and the inserting section 30 can move backward in the lumen in the first modification according to the first embodiment. Also, in this modification, due to the spiral tube section 137a, the thickness of outer tube 131c can be reduced.

### [Second Modification]

### [Spiral Rotary Member 130]

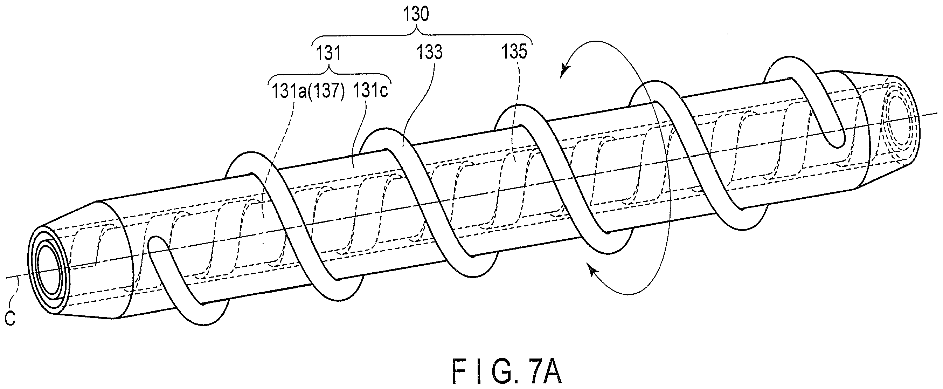

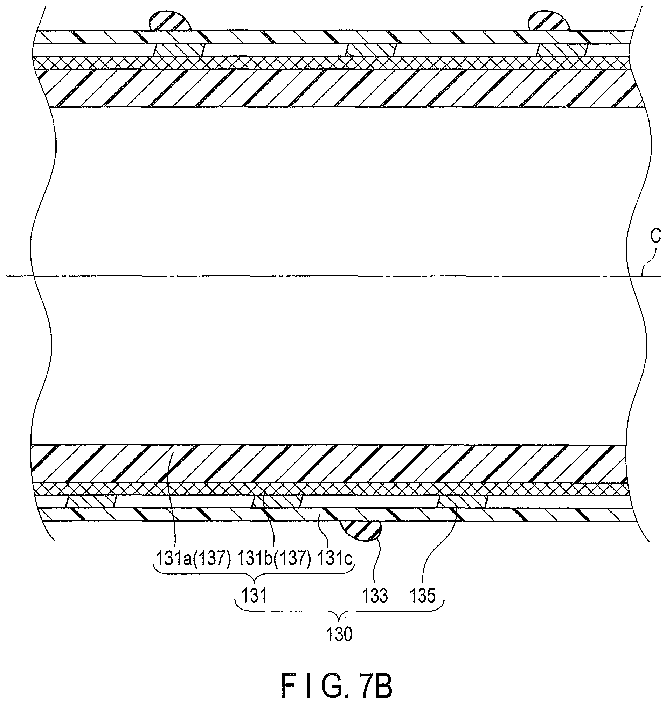

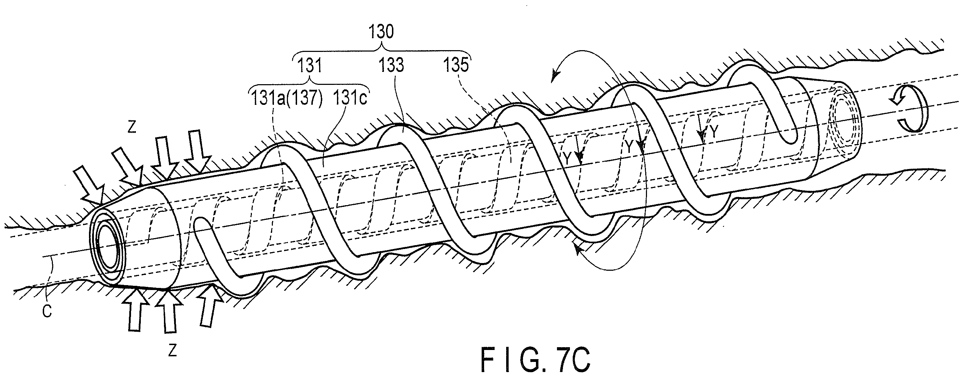

This embodiment is different from the first embodiment in a configuration of a spiral rotary member 130. This embodiment is the same as the first embodiment except for this point, therefore a detailed description thereof is omitted. A description of only the differences between the spiral rotary member 130 according to this embodiment and the first embodiment will be given below, with references to FIG. 7A, FIG. 7B, and FIG. 7C. Note that a reticular tube section 131b is omitted in FIG. 7A to clarify the drawing.

### [Main Body Section 131]

As shown in FIG. 7A and FIG. 7B, in this embodiment, a main body section 131 has, for example, an inner tube 131a that is formed into a substantially circular tube shape so that an inserting section 30 can be inserted into, a reticular tube section 131b that is arranged on an outer side of the inner tube 131a and covers the inner tube 131a that is arranged on an inner side of a spiral tube section 135 and is covered by the spiral tube section 135, and an outer tube 131c that is arranged on an outer side of the spiral tube section 135 and covers the spiral tube section 135.

As shown in FIG. 7B, the inner tube 131a is thicker than the outer tube 131c and functions as an inner thick wall portion of the main body section 131.

Additionally, as shown in FIG. 7B, the outer tube 131c is thinner than the inner tube 131a and functions as an outer thin wall portion of the main body section 131.

In the main body section 131, an outer peripheral surface of the inner tube 131a is bonded to an inner peripheral surface of the spiral tube section 135 through the reticular tube section 131b by, for example , an adhesive (not illustrated).

Furthermore, in this main body section 131, the entire inner peripheral surface of the outer tube 131c is bonded to the entire outer peripheral surface of the spiral tube section 135 by, for example, the adhesive (not illustrated).

The main body section 131 having at least the inner tube 131a can suffice. When the main body section 131 only has the inner tube 131a, the inner tube 131a is directly bonded to the outer peripheral surface of the spiral tube section 135 by, for example, the adhesive (not illustrated). Moreover, when the main body section 131 has the inner tube 131a alone, the fin section 133 is, for example, directly arranged on the outer peripheral surface of the spiral tube section 135.

Also, in this embodiment, the inner tube 131a is bonded to the spiral tube section 135, and the outer tube 131c must be bonded to the spiral tube section 135.

### [Fin Section 133]

As shown in FIG. 7A and FIG. 7B, the fin section 133 is spirally arranged in, for example, the clockwise direction to be wound around the longitudinal axis C like the first embodiment.

### [Spiral Tube Section 135]

As shown in FIG. 7A and FIG. 7B, the spiral tube section 135 according to this embodiment is arranged to be wound around the longitudinal axis C in the direction opposite to the fin section 133, e.g., the counterclockwise direction. The spiral tube section 135 covers the reticular tube section 131b and is covered by the outer tube 131c.

### [Diameter Change Preventing Section 137]

As shown in FIG. 7A and FIG. 7B, when the spiral tube section 135 is wound in the direction opposite to the fin section 133 as in this embodiment, a diameter change preventing section 137 is arranged on the inner side than the spiral tube section 135. In this embodiment, the diameter change preventing section 137 has, e.g., the inner tube 131a that functions as an inner thick wall portion of the main body section 131 and the reticular tube section 131b arranged between the spiral tube section 135 and the inner tube 131a in the radial direction of the spiral rotary member 130. In other words, the inner tube 131a and the reticular tube section 131b also function as the diameter change preventing section 137. It is to be noted that the diameter change preventing section 137 having at least the inner tube 131a can suffice.

In this embodiment, when the spiral tube section 135 wound in the counterclockwise direction rotates in the clockwise direction, torsional force that draws a spiral is applied to the spiral tube section 135. Therefore, the diameter of the spiral tube section 135 is to contract in a Y direction as shown in FIG. 7C. However, the inner peripheral surface of the spiral tube section 135 is bonded to the entire reticular tube section 131b and further bonded to the entire inner tube 131a through the reticular tube section 131b. Therefore, the diameter change preventing section 137 including the inner tube 131a and the reticular tube section 131b supports the spiral tube section 135 against the diameter contraction. Therefore, in FIG. 7C showing a state where the spiral rotary member 130 is inserted in a lumen, the diameter change preventing section 137 including the inner tube 131a prevents the diameter contraction with respect to the spiral tube section 135 that is to radially contract in the Y direction as shown in FIG. 7C. In more detail, the diameter change preventing section 137 applies a counter force in the opposite direction of the Y direction shown in FIG. 7C that prevents the diameter contraction to the spiral tube section 135, thereby preventing the diameter of the spiral tube section 135 from contracting. As a result, when the spiral rotary member 130 rotates in the clockwise direction, the rotating force of the spiral tube section 135 is efficiently transmitted to the entire the entire inner tube 131a that functions as the diameter change preventing section 137. Moreover, when the inner tube 131a rotates in the clockwise direction, the outer tube 131c bonded to the inner tube 131a through the spiral tube section 135 and the reticular tube section 131b also rotates in the clockwise direction, the fin section 133 also rotates in the clockwise direction, and the fin section 133 pulls in the inner wall of the lumen. Therefore, the inserting section 30 moves forward in the lumen.

As described above, the first rotating force is not transmitted to part of the main body section 131, e.g., the proximal end portion of the inner tube 131a from the first rotation member 110. When the spiral tube section 135 is arranged, the first rotating force is transmitted from the first rotation member 110 to the entire main body section 131, e.g., the entire inner tube 131a through the spiral tube section 135. As a result, for example, as shown in FIG. 7C, on the distal end side of the spiral rotary member 130, even if the fin section 133 and the main body section 131 receive resistance Z from the inner wall of the lumen, the rotating force applied to the spiral rotary member 130 is efficiently transmitted to the whole from the proximal end side toward the distal end side of the spiral rotary member 130. Therefore, rotation of the spiral rotary member 130 is prevented from being stopped by the resistance.

### [Function]

When the spiral tube section 135 rotates in the clockwise direction, the diameter of the spiral tube section 135 is to contract, but the diameter change preventing section 137 prevents the diameter of the spiral tube section 135 from contracting. As a result, when the spiral rotary member 130 rotates in the clockwise direction, the rotating force of the spiral tube section 135 is efficiently transmitted to the entire inner tube 131a that functions as the diameter change preventing section 137. Furthermore, when the inner tube 131a rotates in the clockwise direction, the outer tube 131c bonded to the inner tube 131a through the spiral tube section 135 and the reticular tube section 131b also rotates in the clockwise direction, the fin section 133 also rotates in the clockwise direction, and the fin section 133 pull in the inner wall of the lumen. Therefore, the inserting section 30 moves forward in the lumen.

As described above, the first rotating force is not transmitted to part of the main body section 131 like the proximal end portion of the inner tube 131a from the first rotary member 110. Since the spiral tube section 135 is arranged, the first rotating force is transmitted from a position where the first rotating force is transmitted to the end portion of the main body section 131 through the inner tube 131a. Therefore, in comparison to a case where the first rotating force is transmitted to part of the main body section 131, transmissibility of the rotating force to the spiral rotary member 130 is improved in this embodiment. As a result, even if the fin section 133 and the main body section 131 receive resistance from the inner wall of the lumen, rotation of the spiral rotary member 130 can be prevented from being stopped by the resistance, and the spiral rotary member 130 rotates. Furthermore, the fin section 133 pull in the inner wall of the lumen, and the inserting section 30 moves forward in the lumen.

### [Effect]

In this embodiment, when the fin section 133 is wound in the clockwise direction and the spiral tube section 135 is wound in the counterclockwise direction, the same effect as that of the first embodiment can be obtained.

### [Modification 1]

In this embodiment, the description has been given as to the example where the fin section 133 is wound in the clockwise direction and the spiral tube section 135 is wound in the counterclockwise direction.

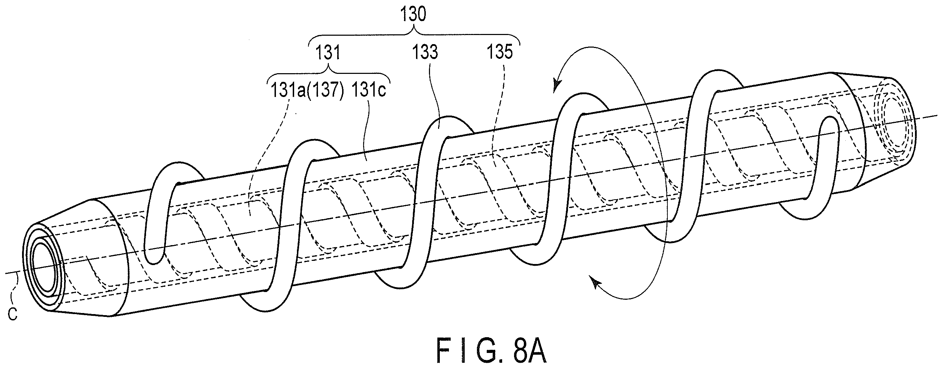

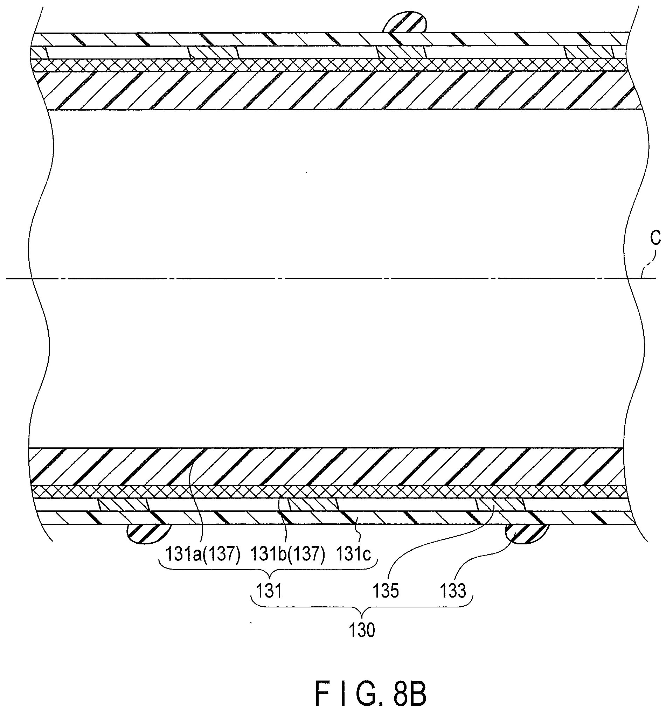

As shown in FIG. 8A and FIG. 8B, and as described in this modification, the fin section 133 is spirally arranged in, for example, the counterclockwise fashion in a direction along which the distal end portion of the main body section 131 is seen from the proximal end portion of the same. Furthermore, in this case, the spiral tube section 135 can be arranged to be wound around the longitudinal axis C in the direction opposite to the fin section 133, i.e., the clockwise direction. As described above, in the direction along which the distal end portion of the main body section 133 is seen from the proximal end portion of the same, the spiral tube section 135 is spirally arranged in the direction opposite to the fin section 133, in other words, the clockwise direction. Note that the reticular tube section 131b is omitted in FIG. 8A to clarify the drawing.

As described above, the spiral tube section 135 may be wound in the direction opposite to the fin section 133. Furthermore, in this case, the diameter change preventing section 137 has the inner tube 131a that functions as the inner thick wall portion of the main body section 131 arranged on the inner side than the spiral tube section 135, and has the reticular tube section 131b arranged between the spiral tube section 135 and the inner tube 131a in the radial direction of the spiral rotary member 130. Likewise, the diameter change preventing section 137 having at least the inner tube 131a can suffice.

As a result, in this modification, it is possible to provide substantially the same effect as that of the first modification according to the first embodiment.

### [Modification 2]

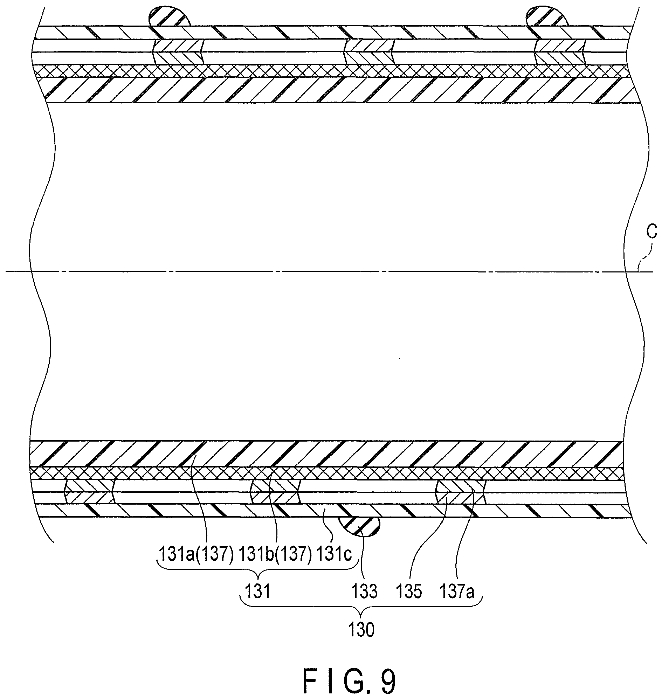

As shown in FIG. 9, the diameter change preventing section 137 also has a diameter change preventing spiral tube section (which will be referred to as a spiral tube section 137a hereinafter) arranged on the inner side of the spiral tube section 135 when the spiral tube section 135 is wound in the direction opposite to the fin section 133. The spiral tube section 137a is spirally arranged to be wound around the longitudinal axis C in the direction opposite to the spiral tube section 135. The spiral tube section 137a has substantially the same configuration as the spiral tube section 135.

The spiral tube section 137a is sandwiched between the spiral tube section 135 and the reticular tube section 131b in the radial direction of the spiral tube section 135.
The spiral tube section 137a is bonded to the spiral tube section 135 and bonded to the inner tube 131a through the reticular tube section 131b.

When the configuration of this modification is combined with the configuration of the second embodiment, the spiral tube section 137a is wound in the clockwise direction as shown in FIG. 9. As described above, in a direction along which the distal end portion of the main body section 131 is seen from the proximal end portion of the same, the spiral tube section 137a is spirally arranged in the same direction as the fin section 133 and the direction opposite to the spiral tube section 135, in other words, the clockwise direction.

Noted that, although not shown, when the configuration of this modification is combined with the configuration of the modification 1 according to the second embodiment, the spiral tube section 137a is wound in the counterclockwise direction.

In this modification, the spiral tube section 137a that functions as the diameter change preventing section 137 can further prevent the diameter contraction of the spiral tube section 135 that rotates in, for example, the clockwise direction in the second embodiment. As a result, in this modification, when the spiral rotary member 130 rotates in the clockwise direction, the rotating force of the spiral tube section 135 can be efficiently transmitted to the entire outer tube 131c and the entire spiral tube section 137a that function as the diameter change preventing section 137. Moreover, in this modification, the fin section 133 can also rotate in the clockwise direction, and the inserting section 30 can move forward in the lumen. Additionally, in this modification, the fin section 133 can also rotate in the counterclockwise direction and the inserting section 30 can move forward in the lumen in the first modification according to the second embodiment. Also, in this modification, the inner tube 131a can be thinned.

### [Summary]

A summary of the first embodiment, each modification of the first embodiment, the second embodiment, and each modification of the second embodiment is as follows.

As described in the first embodiment (FIG. 4A and FIG. 4B) and each modification of the first embodiment (FIG. 5A, FIG. 5B, and FIG. 6), the spiral tube section 135 is arranged to be wound around the longitudinal axis C in the same direction as the fin section 133.

Alternatively, as described in the second embodiment (FIG. 7A and FIG. 7B) and each modification of the second embodiment (FIG. 8A, FIG. 8B, and FIG. 9), the spiral tube section 135 is arranged to be wound around the longitudinal axis C in the direction opposite to the fin section 133.

As described in the first embodiment (FIG. 4A and FIG. 4B) and each modification of the first embodiment (FIG. 5A, FIG. 5B, and FIG. 6), when the spiral tube section 135 is wound in the same direction as the fin section 133, the diameter change preventing section 137 prevents the diameter of the spiral tube section 135 from expanding.

Furthermore, as described in the second embodiment (FIG. 7A and FIG. 7B) and each modification of the second embodiment (FIG. 8A, FIG. 8B, and FIG. 9), when the spiral tube section 135 is wound in the direction opposite to the fin section 133, the diameter change preventing section 137 prevents the diameter of the spiral tube section 135 from contracting.

As described in the first embodiment (FIG. 4A and FIG. 4B) and each modification of the first embodiment (FIG. 5A, FIG. 5B, and FIG. 6), when the spiral tube section 135 is wound in the same direction as the fin section 133, the diameter change preventing section 137 has the outer tube 131c that functions as the outer thick wall portion of the main body section 131 that is arranged on the outer side than the spiral tube section 135. Moreover, in this case, the diameter change preventing section 137 may further have the reticular tube section 131b that is arranged between the spiral tube section 135 and the outer tube 131c.

Additionally, as described in the second embodiment (FIG. 7A and FIG. 7B) and each modification of the second embodiment (FIG. 8A, FIG. 8B, and FIG. 9), when the spiral tube section 135 is wound in the direction opposite to the fin section 133, the diameter change preventing section 137 has the inner tube 131a that functions as the inner thick wall portion of the main body section 131 that is arranged on the inner side than the spiral tube section 135. In this case, the diameter change preventing section 137 may also have the reticular tube section 131b arranged between the spiral tube section 135 and the inner tube 131a.

As described in the second modification of the first embodiment (FIG. 6), when the spiral tube section 135 is wound in the same direction as the fin section 133, the diameter change preventing section 137 has the diameter change preventing spiral tube section 137a that is arranged on the outer side of the spiral tube section 135 and spirally arranged to be wound around the longitudinal axis C in the direction opposite to the spiral tube section 135.

As described in the second modification of the second embodiment (FIG. 9), when the spiral tube section 135 is wound in the direction opposite to the fin section 133, the diameter change preventing section 137 has the diameter change preventing spiral tube section 137a that is arranged on the inner side of the spiral tube section 135 and spirally arranged to be wound around the longitudinal axis C in the direction opposite to the spiral tube section 135.

Furthermore, in each embodiment and each modification, the spiral rotary member 130 has a four-layer structure formed of the inner tube 131a, the reticular tube section 131b, the outer tube 131c, and the spiral tube section 135. When the spiral tube section 135 and the fin section 133 are wound in the same direction each other, to prevent the diameter of the spiral tube section 135 from expanding, the diameter change preventing section 137 may be arranged on the outer side than the spiral tube section 135, and the diameter change preventing section 137 does not have to function as the outermost layer of the spiral rotary member 130. Moreover, the configuration of the spiral rotary member 130 is not specifically restricted.

Additionally, when the spiral tube section 135 and the fin section 133 are wound in the opposite directions each other, to prevent the diameter of the spiral tube section 135 from contracting, the diameter change preventing section 137 may be arranged on the inner side than the spiral tube section 135, and the diameter change preventing section 137 does not have to function as the innermost layer of the spiral rotary member 130. Furthermore, the configuration of the spiral rotary member 130 is not specifically restricted.

It is to be noted that the spiral rotary member 130 functions as the auxiliary insertion and removal device in the foregoing embodiments. An overtube into which the inserting section 30 of the endoscope 20 is inserted may function as an auxiliary insertion and removal device. The overtube has the spiral rotary member 130, and the spiral rotary member 130 has the main body section 131, the fin section 133, the spiral tube section 135, and the diameter change preventing section 137.

## Claims

1. An auxiliary insertion and removal device (130) that is inserted into or removed from a lumen in a state where an inserting section (30) of an endoscope (20) having a longitudinal axis is inserted into and that aids insertion and removal of the inserting section (30) into or from the lumen, the auxiliary insertion and removal device (130) comprising:
a tubular main body section (131) that allows the inserting section (30) to be inserted therein and is rotatable around the longitudinal axis, the tubular main body section (131) including an inner tube (131a), a reticular tube section (131b) having a tubular shape and an outer surface that is formed into a reticular pattern, and an outer tube (131c) that is arranged on an outer side of the reticular tube section (131b) and covers the reticular tube section (131b);
a fin section (133) that is arranged on an outer peripheral surface of the main body section (131) and spirally arranged in a predetermined direction to be wound around the longitudinal axis;
a spiral tube section (135) that is arranged on the main body section (131) and arranged to be wound around the longitudinal axis in the same direction as the fin section (133) or a direction opposite to the fin section (133); and
a diameter change preventing section (137) having the outer tube (131c) that is arranged in the main body section (131) and that functions as an outer thick wall portion of the main body section (131), the diameter change preventing section (137) being configured to prevent a diameter of the spiral tube section (135) from expanding when the spiral tube section (135) is wound in the same direction as the fin section (133) and the main body section (131) is rotated in the predetermined direction, or to prevent the diameter of the spiral tube section (135) from contracting when the spiral tube section (135) is wound in the direction opposite to the fin section (133) and the main body section (131) is rotated in the predetermined direction,
wherein the inner tube (131a) of the main body section (131) is arranged on an inner side of the spiral tube section (135) and is covered by the spiral tube section (135), and the reticular tube section (131b) of the main body section (131) is arranged on an outer side of the spiral tube section (135) and covers the spiral tube section (135),
**characterized in that**
the diameter change preventing section (137) has a diameter change preventing spiral tube section (137a) that is arranged on the outer side of the spiral tube section (135) when the spiral tube section (135) is wound in the same direction as the fin section (133), or is arranged on the inner side of the spiral tube section (135) when the spiral tube section (135) is wound in the direction opposite to the fin section (133),
the diameter change preventing spiral tube section (137a) is arranged to be wound around the longitudinal axis in a direction opposite to the spiral tube section (135), and is sandwiched between the spiral tube section (135) and the reticular tube section (131b) in the radial direction of the spiral tube section (135), and is bonded to the spiral tube section (135) and to the outer tube (131c) through the reticular tube section (131b).

2. The auxiliary insertion and removal device (130) according to claim 1,
wherein the diameter change preventing section (137) has the outer thick wall portion (131c) arranged on the outer side than the spiral tube section (135) when the spiral tube section (135) is wound in the same direction as the fin section (133), and
the diameter change preventing section (137) has an inner thick wall portion (131a) arranged on the inner side than the spiral tube section (135) when the spiral tube section (135) is wound in the direction opposite to the fin section (133).

3. The auxiliary insertion and removal device (130) according to claim 2,
wherein the diameter change preventing section (137) further has the reticular tube section (131b) arranged between the outer thick wall portion (131c) and the spiral tube section (135) when the spiral tube section (135) is wound in the same direction as the fin section (133), and
the diameter change preventing section (137) further has the reticular tube section (131b) arranged between the inner thick wall portion (131a) and the spiral tube section (135) when the spiral tube section (135) is wound in the direction opposite to the fin section (133).

4. An endoscope (20) having the auxiliary insertion and removal device (130) according to claim 1.

## Patentansprüche

1. Hilfsvorrichtung zur Einsetzung und Entnahme (130), die in einem Zustand in ein Lumen eingesetzt oder daraus entnommen wird, in dem ein Einsetzabschnitt (30) eines Endoskops mit einer Längsachse eingesetzt wird, und die das Einsetzen und die Entnahme des Einsetzabschnitts (30) in das oder aus dem Lumen unterstützt, wobei die Hilfsvorrichtung zur Einsetzung und Entnahme (130) umfasst:
einen röhrenförmigen Hauptkörperabschnitt (131), der es ermöglicht, dass der darin einzusetzende Einsetzabschnitt (30) um die Längsachse rotiert werden kann, wobei der röhrenförmige Hauptkörperabschnitt (131) eine Innenröhre (131a), einen netzartigen Röhrenabschnitt (131b) mit einer röhrenförmigen Form und einer Außenfläche, die in einem Netzmuster gebildet ist, und eine Außenröhre (131c) umfasst, die auf einer Außenseite des netzartigen Röhrenabschnitts (131b) angeordnet ist und den netzartigen Röhrenabschnitt (131b) abdeckt;
einen Lamellenabschnitt (133), der auf einer Außenumfangsfläche des Hauptkörperabschnitts (131) angeordnet ist und in einer vorbestimmten Richtung spiralförmig angeordnet ist, um um die Längsachse gewickelt zu sein;
einen spiralförmigen Röhrenabschnitt (135), der auf dem Hauptkörperabschnitt (131) angeordnet ist und angeordnet ist, um in derselben Richtung wie der Lamellenabschnitt (133) oder in eine Richtung entgegen dem Lamellenabschnitt (133) um die Längsachse gewickelt zu sein; und
einen Durchmesseränderungsverhinderungsabschnitt (137) mit der Außenröhre (131c), die in dem Hauptkörperabschnitt (131) angeordnet ist und die als ein äußerer Dickwandabschnitt des Hauptkörperabschnitts (131) fungiert, wobei der Durchmesseränderungsverhinderungsabschnitt (137) dazu ausgelegt ist, zu verhindern, dass sich ein Durchmesser des spiralförmigen Röhrenabschnitts (135) aufweitet, wenn der spiralförmige Röhrenabschnitt (135) in dieselbe Richtung gewickelt ist wie der Lamellenabschnitt (133) und der Hauptkörperabschnitt (131) in die vorbestimmte Richtung rotiert wird, oder zu verhindern, dass der Durchmesser des spiralförmigen Röhrenabschnitts (135) kleiner wird, wenn der spiralförmige Röhrenabschnitt in die Richtung entgegen dem Lamellenabschnitt (133) gewickelt ist und der Hauptkörperabschnitt (131) in die vorbestimmte Richtung rotiert wird,
wobei die Innenröhre (131a) des Hauptkörperabschnitts (131) auf einer Innenseite des spiralförmigen Röhrenabschnitts (135) angeordnet ist und von dem spiralförmigen Röhrenabschnitt (135) abgedeckt wird, und der netzartige Röhrenabschnitt (131b) des Hauptkörperabschnitts (131) auf einer Außenseite des spiralförmigen Röhrenabschnitts (135) angeordnet ist und den spiralförmigen Röhrenabschnitt (135) abdeckt,
**dadurch gekennzeichnet, dass**
der Durchmesseränderungsverhinderungsabschnitt (137) einen Durchmesseränderungsverhinderungsspiralröhrenabschnitt (137a) aufweist, der auf der Außenseite des spiralförmigen Röhrenabschnitts (135) angeordnet ist, wenn der spiralförmige Röhrenabschnitt (135) in derselben Richtung gewickelt ist wie der Lamellenabschnitt (133), oder auf der Innenseite des spiralförmigen Röhrenabschnitts (135) angeordnet ist, wenn der spiralförmige Röhrenabschnitt (135) in die Richtung entgegen dem Lamellenabschnitt (133) gewickelt ist,
der Durchmesseränderungsverhinderungsspiralröhrenabschnitt (137a) angeordnet ist, um um die Längsachse in eine Richtung entgegen dem spiralförmigen Röhrenabschnitt (135) gewickelt zu sein, und zwischen den spiralförmigen Röhrenabschnitt (135) und den netzartigen Röhrenabschnitt (131b) in der Radialrichtung des spiralförmigen Röhrenabschnitts (135) eingeschoben ist, und mit dem spiralförmigen Röhrenabschnitt (135) und der Außenröhre (131c) über den netzartigen Röhrenabschnitt (131b) verbunden ist.

2. Hilfsvorrichtung zur Einsetzung und Entnahme (130) nach Anspruch 1,
wobei der Durchmesseränderungsverhinderungsabschnitt (137) den äußeren Dickwandabschnitt (131c) auf der Außenseite des spiralförmigen Röhrenabschnitts (135) angeordnet aufweist, wenn der spiralförmige Röhrenabschnitt (135) in derselben Richtung gewickelt ist wie der Lamellenabschnitt (133), und
wobei der Durchmesseränderungsverhinderungsabschnitt (137) den inneren Dickwandabschnitt (131c) auf der Innenseite des spiralförmigen Röhrenabschnitts (135) angeordnet aufweist, wenn der spiralförmige Röhrenabschnitt (135) in die Richtung entgegen dem Lamellenabschnitt (133) gewickelt ist.

3. Hilfsvorrichtung zur Einsetzung und Entnahme (130) nach Anspruch 2,
wobei der Durchmesseränderungsverhinderungsabschnitt (137) ferner den netzartigen Röhrenabschnitt (131b) zwischen dem äußeren Dickwandabschnitt (131c) und dem spiralförmigen Röhrenabschnitt (135) aufweist, wenn der spiralförmige Röhrenabschnitt (135) in derselben Richtung gewickelt ist wie der Lamellenabschnitt (133), und
wobei der Durchmesseränderungsverhinderungsabschnitt (137) ferner den netzartigen Röhrenabschnitt (131b) zwischen dem inneren Dickwandabschnitt (131a) und dem spiralförmigen Röhrenabschnitt (135) aufweist, wenn der spiralförmige Röhrenabschnitt (135) in die Richtung entgegen dem Lamellenabschnitt (133) gewickelt ist.

4. Endoskop (20) mit der Hilfsvorrichtung zur Einsetzung und Entnahme (130) nach Anspruch 1.

## Revendications

1. Dispositif d'insertion et d'extraction auxiliaire (130) qui est inséré dans ou extrait d'une lumière dans un état dans lequel une section d'insertion (30) d'un endoscope (20) ayant un axe longitudinal est insérée, et qui facilite l'insertion et l'extraction de la section d'insertion (30) dans ou à partir de la lumière, le dispositif d'insertion et d'extraction auxiliaire (130) comprenant :
une section corps principal tubulaire (131) qui permet à la section d'insertion (30) d'être insérée dans celle-ci et est apte à tourner autour de l'axe longitudinal, la section corps principal tubulaire (131) comprenant un tube interne (131a), une section tube réticulaire (131b) ayant une forme tubulaire et une surface externe qui est formée dans un motif réticulaire, et un tube externe (131c) qui se trouve sur un côté externe de la section tube réticulaire (131b) et recouvre la section tube réticulaire (131b) ;
une section ailette (133) qui est située sur une surface périphérique externe de la section corps principal (131) et disposée en spirale dans une direction prédéterminée afin d'être enroulée autour de l'axe longitudinal ;
une section tube en spirale (135) qui est située sur la section corps principal (131) et destinée à être enroulée autour de l'axe longitudinal dans la même direction que la section ailette (133) ou une direction opposée à la section ailette (133) ; et
une section de prévention de changement de diamètre (137) ayant le tube externe (131c) qui est situé dans la section corps principal (131) et qui agit comme partie de paroi épaisse externe de la section corps principal (131), la section de prévention de changement de diamètre (137) étant configurée pour empêcher un diamètre de la section tube en spirale (135) de s'étendre lorsque la section tube en spirale (135) est enroulée dans la même direction que la section ailette (133) et que la section corps principal (131) est tournée dans la direction prédéterminée, ou pour empêcher le diamètre de la section tube en spirale (135) de se contracter lorsque la section tube en spirale (135) est enroulée dans la direction opposée à la section ailette (133) et que la section corps principal (131) est tournée dans la direction prédéterminée, le tube interne (131a) de la section corps principal (131) étant situé sur un côté interne de la section tube en spirale (135) et étant recouvert par la section tube en spirale (135), et la section tube réticulaire (131b) de la section corps principal (131) étant située sur un côté externe de la section tube en spirale (135) et recouvrant la section tube en spirale (135),
**caractérisé par le fait que**
la section de prévention de changement de diamètre (137) a une section tube en spirale de prévention de changement de diamètre (137a) qui est située sur le côté externe de la section tube en spirale (135) lorsque la section tube en spirale (135) est enroulée dans la même direction que la section ailette (133), ou est située sur le côté interne de la section tube en spirale (135) lorsque la section tube en spirale (135) est enroulée dans la direction opposée à la section ailette (133),
la section tube en spirale de prévention de changement de diamètre (137a) est agencée pour être enroulée autour de l'axe longitudinal dans une direction opposée à la section tube en spirale (135), et est prise en sandwich entre la section tube en spirale (135) et la section tube réticulaire (131b) dans la direction radiale de la section tube en spirale (135), et est liée à la section tube en spirale (135) et au tube externe (131c) par l'intermédiaire de la section tube réticulaire (131b).

2. Dispositif d'insertion et d'extraction auxiliaire (130) selon la revendication 1, dans lequel la section de prévention de changement de diamètre (137) a la partie de paroi épaisse externe (131c) située sur le côté externe de la section tube en spirale (135) lorsque la section tube en spirale (135) est enroulée dans la même direction que la section ailette (133), et
la section de prévention de changement de diamètre (137) a une partie de paroi épaisse interne (131a) située sur le côté interne de la section tube en spirale (135) lorsque la section tube en spirale (135) est enroulée dans la direction opposée à la section ailette (133).

3. Dispositif d'insertion et d'extraction auxiliaire (130) selon la revendication 2, dans lequel la section de prévention de changement de diamètre (137) a en outre la section tube réticulaire (131b) située entre la partie de paroi épaisse externe (131c) et la section tube en spirale (135) lorsque la section tube en spirale (135) est enroulée dans la même direction que la section ailette (133), et
la section de prévention de changement de diamètre (137) a en outre la section tube réticulaire (131b) située entre la partie de paroi épaisse interne (131a) et la section tube en spirale (135) lorsque la section tube en spirale (135) est enroulée dans la direction opposée à la section ailette (133).

4. Endoscope (20) ayant le dispositif d'insertion et d'extraction auxiliaire (130) selon la revendication 1.
